# EUROPEAN PATENT APPLICATION

(11) **EP 2 719 758 A1**
(43) Date of publication of application: **16.04.2014**
(21) Application number: 12187783.1
(22) Date of filing: 09.10.2012
(51) Int. Cl.: C12N 9/24, C12N 9/42

(54) **Cellulase assay**

(71) Applicant: Megazyme IP Limited, Bray Wicklow (IE)
(72) Inventor: McCleary, Barry Vincent, Co. Wicklow (IE); Mangan, David Peter, Co. Dublin (IE)
(74) Representative: Gates, Marie Christina Esther

(57) **Abstract**

The invention involves the development of a substrate and assay procedure for the scientific measurement of cellulase in admixture with other enzymes active on cellulose or 1,4-β-D-gluco-oligosaccharides, or in various levels of purity of the cellulase. This substrate being characterised by the fact that it contains at least 2 glucose units linked β-1,4-, with the reducing end glucose unit linked β- or α- to a compound which exhibits an optically measurable change on cleavage of the bond between it and the adjacent D-glucosyl residue and the non-reducing terminal D-glucosyl unit is covalently linked to a blocking substituent which inhibits cleavage by *exo*-acting enzymes of the bond between the terminal and non-reducing end, penultimate D-glucose unit.

## Description

### Field of the Invention

The invention relates to methods for the specific measurement of *endo-*1,4*-*β*-*D-glucanase (hereafter called cellulase) and to assay kits for such measurement. Such methods and kits find use in the measurement of cellulase in the analysis of microbial fermentation products, in industrial enzyme preparations and other biological materials containing cellulase enzyme activity and in standardising cellulase for biofuel production.

### Background to the Invention

Numerous assays have been described for the specific and non-specific measurement of cellulase in biological fluids including those based on the measurement of increase in reducing sugars on hydrolysis of cello-oligosaccharides, chemically modified or non-modified cellulose or 1,3;1,4-β-D-glucan (McCleary, *et al.,* 2012); increase in dyed fragments soluble in an aqueous/organic solvent on hydrolysis of soluble dyed polysaccharides; increase in water soluble, dyed fragments or hydrolysis of insoluble, dyed and crosslinked polysaccharides (McCleary, *et al.,* 2012). Japanese Patent JP02083390 describes the preparation and use of nitrophenyl-cello-oligosacchardes for the measurement of cellulase, and such nitrophenyl-cello-oligosaccharides have been used by Rahman *et al.* (2002) in studies on the action pattern of *Thermatoga maritima endo*-1,4-β-glucanase (cellulase). However, since cellulase occurs in admixture with other enzymes which are active on nitrophenyl-cello-oligosaccharides, namely, β-glucosidase and *exo*-1,4-β-D-glucanases, the described substrates cannot be used for the specific and quantitative measurement of cellulase in fermentation broths and industrial enzyme preparations.

Blair (1988) describes a procedure for the measurement of α-amylase in biological fluids using end-blocked nitrophenyl malto-oligosaccharides. However, since the date of that invention, no parallel substrate or assay procedure has been developed or published for measurement of cellulase, although such a compound or product would be highly desirable.

### Object of the Invention

It is thus an object of the present invention to provide a method and kit for the determination and measurement of cellulase where it occurs in admixture with other enzymes which are active on nitrophenyl-cello-oligosaccharides, such as β-glucosidase and *exo*-1,4-β-D-glucanases. A further object is to provide methods and kits which can be used for the specific and quantitative measurement of cellulase in fermentation broths and industrial enzyme preparations and the like. A further object of the invention is to provide methods and kits for cellulase measurement and determination which are cheap to produce, easy to conduct and reliable.

### Summary of the Invention

According to the present invention there is provided a reagent kit for measurement of cellulase comprising, an oligosaccharide substrate for assay of cellulase, the said substrate containing at least 2 glucose units linked by a 1,4- β-linkage, with the reducing-end glucose of the oligosaccharide linked via a bond cleavable by β- or α-glucosidase, to a label which exhibits a measurable change upon cleavage of the bond, and the non-reducing glucose unit being linked to a chemical blocking substituent which inhibits cleavage by *exo*-enzymes of the bond between the non-reducing end glucose unit and the adjacent glucose unit; and an *exo*-enzyme (β-glucosidase) capable of cleaving the bonds between the non-blocked glucose residues and the label.

This invention includes the steps involved in modifyng the cello-oligosaccharides to provide end-blocked colourimetric/fluorimetric substrates for cellulase as well as methods for dissolution of the end-blocked nitrophenyl-cello-oligosaccharides and optimised ancilliary enzyme(s) for use with the oligosaccharide subtrates.

In general, this invention involves the development of a substrate and assay procedure for the scientific measurement of cellulase in admixture with other enzymes active on cellulose or 1,4-β-D-gluco-oligosaccharides, or in various levels of purity of the cellulase. This substrate is characterised by the fact that it contains at least 2 glucose units linked β-1,4-, with the reducing end glucose unit linked β- or α- to a compound which exhibits an optically measurable change on cleavage of the bond between it and the adjacent D-glucosyl residue. Furthermore, and critically, the non-reducing terminal D-glucosyl unit is covalently linked to a blocking substituent which inhibits cleavage by *exo*-acting enzymes of the bond between the terminal and non-reducing end, penultimate D-glucose unit. This blocking group itself, not being susceptible to hydrolysis by enzymes in the mixture being analysed, e.g. esterase or lipase enzymes. The invention also embodies a method for solubilising said end-blocked, nitrophenyl cello-oligosaccharide allowing its use in enzyme assay procedures.

Reaction is initiated by mixing solubilised substrate, *exo*-enzyme capable of hydrolysing the link between the label and α- or β-linked glucose, and the *endo*-acting cellulase. Reaction is followed automatically or manually depending on the nature of the label used and the pH of the reaction mixture.

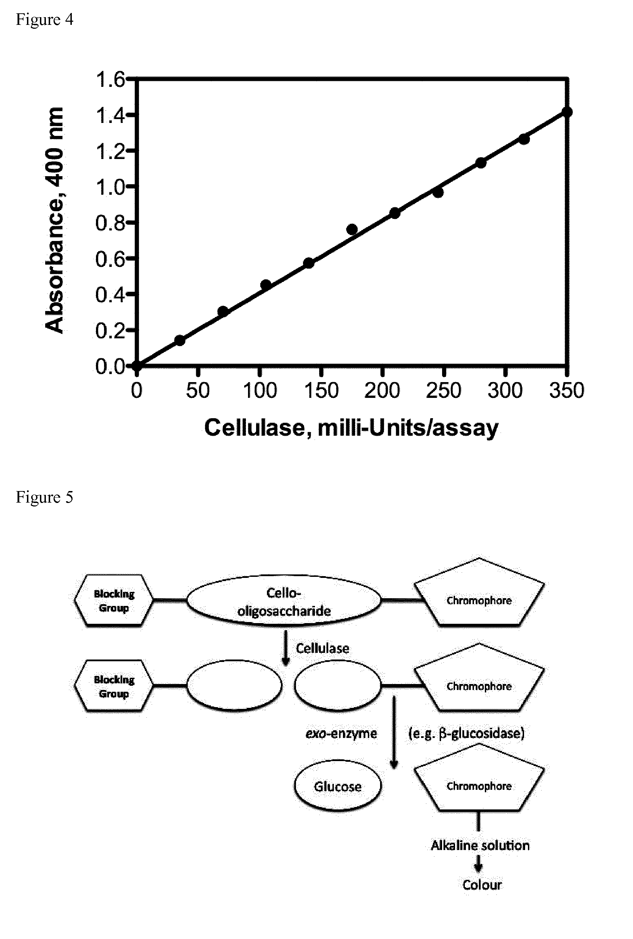

In preferred embodiments, the label is a chromophore, a fluorophore, a chemiluminescent substituent, or a bioluminescent substituent; the *exo*-enzyme is β-glucosidase, but may also be a mixture of β-glucosidase) and α-glucosidase. Preferred labels include chloro-*p*-nitrophenol ClPNP), *p*-nitrophenol (PNP), *o*-nitrophenol (chromophores), coumarin derivatives such as 4-methylumbelliferone (a fluorophore), and luciferin (a chemiluminescent substituent). Preferably, the substrate has six or fewer glucose units, and most preferably has two, three or four. Preferred blocking substituents are acetals or ketals, e.g. benzylidene. The reagents used in the assay are preferably provided in the form of a reagent kit, the reagents preferably being mixed at the time of assay. The assay of the invention works according to the general scheme shown in Figure 5. In the scheme illustrated in Figure 5, the blocking group prevents *exo*-enzymes from breaking down the substrate, so that in the absence of cellulase there will be no colour change. Cellulase, an *endo*-enzyme, cleaves internal β-1,4 bonds in the substrate, producing smaller fragments which can be acted on by the *exo*-enzyme, which causes the ultimate release of the chromophore.

The invention also provides a process for the dissolution of the end-blocked *p-*nitrophenyl cello-oligosaccharide or end-blocked Cl-*p*-nitrophenyl cello-oligosaccharide, either of which are sparingly soluble in water or buffer solutions, comprising use of a chaotropic solvent. Preferably, the solute inhibits neither the cellulase being measured nor the enzymes β-glucosidase) and α-glucosidase used in the assay. An example of a suitable solvent is dimethyl sulphoxide.

The assay of the invention produces a linear, or near-linear result, i.e. optical density (400 nm) measurements are proportional to cellulase concentration and/or the time of incubation, rendering the assay highly amenable to automation. In addition, the components of the assay (substrate dissolved in dimethyl sulphoxide and potassium chloride solution and β-glucosidase) can be combined before use and show good stability over several hours at room temperature, because the blocking group prevents degradation of the substrate by β-glucosidase.

### Brief Description of the Drawings

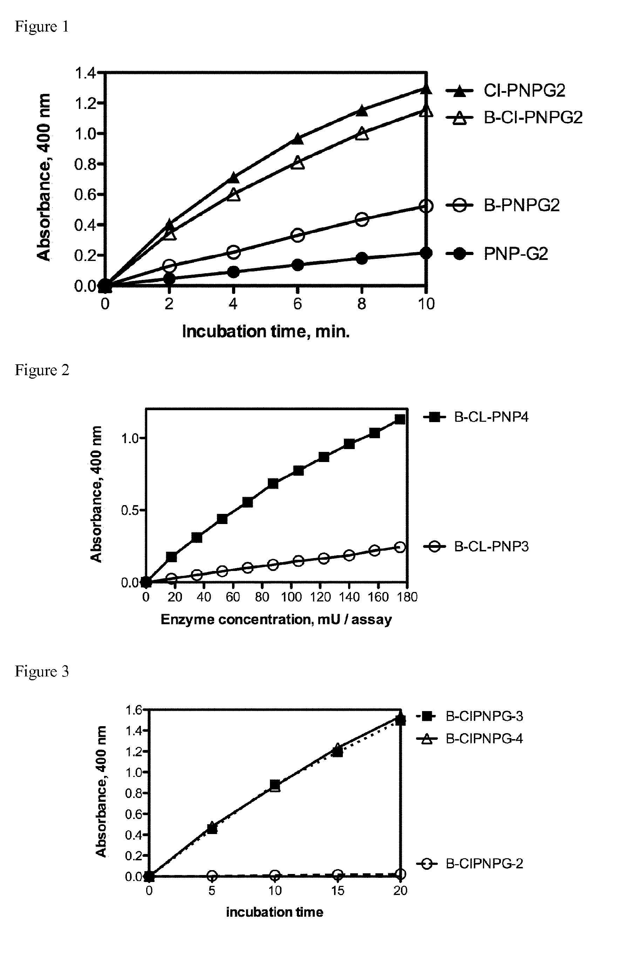

**Figure 1****.** is a graph of absorbance (400 nm) vs. time for the action of a cellulase from *Trichoderma longibrachiatum* (1500 milli-International Units on CM-Cellulose per assay) on *p*-nitrophenyl cellobiose (PNP-G2), 2-chloro-*p*-nitrophenyl cellobiose (ClPNP-G2), end-blocked *p*-nitrophenyl cellobiose (BPNP-G2) and end-blocked 2-chloro *p*-nitrophenyl cellobiose (BClPNP-G2) at 40°C and pH 4.5 using an assay of the invention.
**Figure 2****.** is a graph of absorbance (400 nm) vs. enzyme concentration for the action of a cellulase from *Aspergillus niger* (17.5 to 175 milli-International Units on CM-Cellulose per assay) on end-blocked 2-chloro-*p*-nitrophenyl cellotriose (BClPNP-G3) and end-blocked 2-chloro-*p*-nitrophenyl cellotetraose (BClPNP-G4) at 40°C and pH 4.5 using an assay of the invention.
F**igure 3.** is a graph of absorbance (400 nm) vs. time for hydrolysis of end-blocked 2-chloro-*p*-nitrophenyl cellobiose (BClPNP-G2), end-blocked 2-chloro-*p-*nitrophenyl cellotriose (BClPNP-G3) and end-blocked 2-chloro-*p*-nitrophenyl cellotetraose (BClPNP-G4) *Trichoderma longibrachiatum* cellulase (30 milli-International Units on CM-Cellulose per assay) at 40°C and pH 4.5 using an assay of the invention.
**Figure 4****.** is a graph of absorbance (400 nm) vs. enzyme concentration for the action of a cellulase from *Thermatoga maritima* (35 to 350 milli-International Units on CM-Cellulose at 80°C per assay) on end-blocked 2-chloro-*p*-nitrophenyl cellotetraose (BClPNP-G4) at 80°C and pH 6.5 using an assay of the invention.
**Figure 5****.** is a diagrammatic representation of the assay scheme of the invention. The *endo-*acting celluase cleaves the glycosidic bond between two glucosyl units and/or the bond between the terminal glucosyl unit and 2-chloro *p*-nitrophenyl releasing an end-blocked cello-oligosaccharide and an unblocked 2-chloro-*p*-nitrophenyl cello-oligosaccharide fragment, or free 2-chloro-*p*-nitrophenol. The β-glucosidase) (and/or α-glucosidase) in the assay mixture immediately hydrolyses the unblocked 2-chloro-*p-*nitrophenyl cello-oligosaccharide released, giving glucose and free 2-chloro-*p-*nitrophenol.

### Detailed Description of the Drawings

### STRUCTURE AND SYNTHESIS OF SUBSTRATE

The oligosaccharide portion of the substrate can generally be obtained commercially, or can be produced by acid or enzymic hydrolysis of cellulose or cellulose derivatives, as is known to those skilled in the art.

The label portion, preferably a chromophore such as 2-chloro-*p*-nitrophenol, *p-*nitrophenol or *o*-nitrophenol, or a fluorophore such as 4-methylumbelliferone, can be attached to the reducing-end glucose unit using standard techniques, e.g. those described in Planas *et al.* (1998) and Dess *et al.* (1981) and cellobiose labelled with 2-chloro-*p-*nitrophenol is commercially available, e.g. from Carbosynth.

The blocking group can be any substituent which prevents *exo*-enzymes from breaking down the substrate. The blocking group works by creating a terminal glucose unit no longer capable of fitting the active site of the *exo*-enzyme. Thus, the size and chemical composition of the blocking group are not critical; all that is required is that interaction between the enzyme and the substrate is prevented. Any substituent bonded to C2, C3, C4 or C6 of the terminal glucose unit that blocks the action of *exo*-enzymes can be used.

One class of blocking groups can replace a hydrogen in the hydroxyl group of C6 of the terminal glucose unit. Suitable such groups include, e.g. carboxylic acid esters (e.g. acetyl or benzoyl); phosphate esters; sulfonate esters (e.g. toluenesulfonyl or methanesulfonyl); ethers (e.g. benzyl, silyl and triphenylmethyl).

Alternatively, the blocking group can be an acetal or ketal blocking group, i.e. a group which blocks the C4 and C6 hydroxyls of the terminal glucose unit:

Synthesis of a blocked substrate for use in the assay of the invention can be carried out according to the following general scheme; the illustrated chromophore is either *p*-nitrophenol or 2-Cl-*p*-nitrophenol, the blocking group is benzylidene, and there are n + 1 glucose units, where 1 ≤ n ≤ 5:

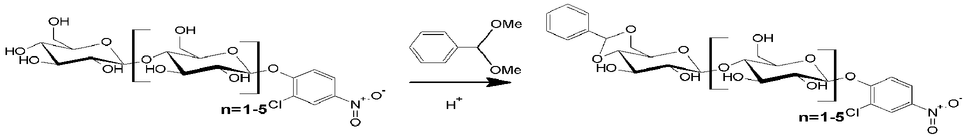

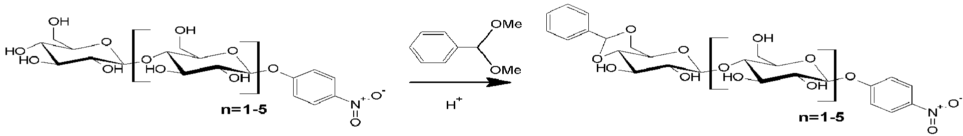

A substrate containing four glucose units, blocked with benzylidene and labelled with 2 chloro-*p*-nitrophenol, has the formula O-(4,6-O-benzylidene-β-D-glucopyranosyl)-(1-4)-O-β-D-glucopyranosyl)-(1-4)-O-β-D-glucopyranosyl)-1-O-(2-chloro-4-nitrophenol-O-α-D-glucopyranoside) **(1),** and the structure:

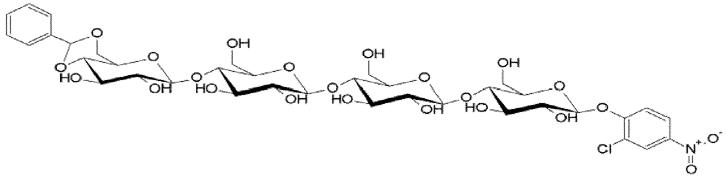

Compound **(1)** was synthesised as follows: To a solution of 2-Cl-*p*-nitrophenol cellotetraoside (1 g, 1.217 mmol) and *p*-toluenesulfonic acid monohydrate (69 mg, 0.365 mmol) in anhydrous dimethylformamide (30 mL) under an argon atmosphere containing activated 4A molecular sieves (200 mg) was added benzaldehyde dimethylacetal (917 µL, 6.083 mmol). The reaction was heated to 50°C and stirred for 14 hours. After this time, TLC analysis indicated reaction completion. Triethylamine (69 µL, 0.494 mmol) was added and the reaction cooled to room temperature. The crude reaction mixture was absorbed onto silica gel and semi-purified by flash chromatography. The fractions obtained containing the desired product were combined and further purified by recrystallization from a 2: 1 mixture of acetonitrile and water.

The product of this reaction had the formula O-(4,6-O-benzylidene-β-D-glucopyranosyl)-(1-4)-O-(β-D-glucopyranosyl)-(1-4)-O-(β-D-glucopyranosyl)-1-O-(2-chloro-4-nitrophenol-O-β-D-glucopyranoside) **(1).** The desired product was obtained as a white solid (390 mg, 0.429 mmol, 35%).

### CELLULASE (endo-1,4-β-GLUCANASE) ASSAY

### Substrate Dissolution

Prepare substrate solution by dissolving end-blocked *p*-nitrophenyl cello-oligosaccharides or end-blocked 2-Cl-*p*-nitrophenyl cello-oligosaccharide (DP 2, 3 or 4) in 2 mL of dimethyl sulphoxide to obtain a concentration of 12.5 mM. Add 3 mL of 170 mM potassium chloride solution and mix to give a final substrate concentration of 5 mM. Prepare this solution fresh before use, or warm to 40°C before use to ensure complete dissolution.

### Manual Assay Format

Add an aliquot (0.1 mL) of the substrate solution to the bottom of a 16 x 120 mm glass test tube, followed by 0.05 mL of pure β-glucosidase. Incubate tubes for 2 min at the selected reaction temperature and initiate the reaction by adding 0.1 mL of sample solution containing cellulase in 200 mM buffer solution of the desired pH (usually in the range of 4.5 to 8.0). After incubation for the desired time (e.g. 10 min), terminate the reaction by adding 3 mL of 2 % w/v Tris buffer (pH 9) or tri-sodium phosphate solution (pH 11). Mix the tube contents and measure the absorbance at 400 nm against a reaction blank.

### Microplate Assay Format

Add an aliquot (0.02 mL) of the substrate solution to the bottom of the micro-well followed by 0.02 mL of pure β-glucosidase. Mix well, and after 1 min at 40°C, initiate the reaction by adding 0.02 mL of sample solution containing cellulase in 200 mM buffer solution of the desired pH (usually in the range of 4.5 to 8.0). After incubation for the desired time (e.g. 10 min), terminate the reaction by adding 0.3 mL of 2 % w/v tris buffer (pH 9) or tri-sodium phosphate solution (pH 11). Mix the tube contents and measure the absorbance at 400 nm against a reaction blank. Run standard cellulase preparations concurrently.

### REAGENT KIT

The reagents used in the assay method of this invention are preferably provided in the form of a reagent kit which includes:
1. Blocked, labelled substrate (preferably containing 3, 4, or 5 glucose units) (either as a powder or as a stabilised solution ready for use).
2. Pure β-glucosidase) (either as a stabilised solution or lyophilised powder).
3. A cellulase control preparation (either as a stabilised solution or lyophilised powder).

### REFERENCES

Planas, A., Abel, M., Millet, J., Palasi, Ó., Pallarés, C. and Viladot, J.L. Synthesis of aryl 3-O-β-cellobiosyl-β-D-glucopyranosides for reactivity studies of 1,3-1,4-β-glucanases. Carbohydrate Research (1998) 310:53-64.
Dess, D., Kleine, H.P., Weinberg, D.V., Kaufman, R.J. and Sidhu, R.S. Phase-transfer catalysed synthesis of acetylated aryl β-D-glucopyranosides and aryl β-D-galactopyranosides. Synthesis (1981) 883-885.
McCleary, B.V., McKie, V. and Draga, A. Measurement of 1,4-β-glucanase. Methods in Enzymology, (2012) 510:1-17.
Rahman, Md.M, Bhuiyan, S.H., Nirasawa, S, Kitaoka, M and Hayashi, K. Characterisation of an endo-β-1,4-glucanase of Thermatoga maritima expressed in Escherichia coli. J. Appl. Glycoscience (2002) 49:487-495.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A reagent kit for measurement of cellulase comprising, an oligosaccharide substrate for assay of cellulase, the said substrate containing at least 2 glucose units linked by a 1,4- β-linkage, with the reducing-end glucose of the oligosaccharide linked via a bond cleavable by β- or α-glucosidase, to a label which exhibits a measurable change upon cleavage of the bond, and the non-reducing glucose unit being linked to a chemical blocking substituent which inhibits cleavage by *exo*-enzymes of the bond between the non-reducing end glucose unit and the adjacent glucose unit; and an *exo-*enzyme (β-glucosidase)) capable of cleaving the bonds between the non-blocked glucose residues and the label.

2. A kit as claimed in claim 1 further comprising a second *exo*-enzyme (α-glucosidase) if the oligosaccharide is α-linked to the label.

3. A kit as claimed in claim 1 or 2 wherein the measurable change upon cleavage of the bond is optically measurable.

4. A kit as claimed in any preceding claim wherein the label is a chromophore, a fluorophore, a chemiluminescent substituent, or a bioluminescent substituent.

5. A kit as claimed in claim 4 wherein the label is selected from chloro-*p-*nitrophenol ClPNP), *p*-nitrophenol (PNP), *o*-nitrophenol, coumarin derivatives such as 4-methylumbelliferone, and luciferin.

6. A kit as claimed in any preceding claim wherein the *exo*-enzyme is β-glucosidase or a mixture of β-glucosidase) and α-glucosidase.

7. A kit as claimed in any preceding claim wherein the substrate has six or fewer glucose units.

8. A kit as claimed in claim 7 wherein the substrate has two, three or four glucose units.

9. A kit as claimed in any preceding claim wherein the blocking substituents are acetals or ketals, e.g. benzylidene.

10. A method for the measurement of cellulase in a sample comprising incubating the sample with an oligosaccharide substrate and with an exo-enzyme, the substrate containing at least 2 glucose units linked by a 1,4- β-linkage, with the reducing-end glucose of the oligosaccharide linked via a bond cleavable by β- or α-glucosidase, to a label which exhibits a measurable change upon cleavage of the bond, the exo-enzyme being capable of cleaving the bond between the reducing-end glucose unit and the label, and measuring a change between the sample and a control, on cleavage of the bond, the change indicating the amount of cellulase in the sample.

11. A process for the dissolution of the end-blocked *p*-nitrophenyl cello-oligosaccharide or end-blocked Cl-*p*-nitrophenyl cello-oligosaccharide, either of which are sparingly soluble in water or buffer solutions, comprising use of a chaotropic solvent.

12. A process as claimed in claim 11 wherein the solute inhibits neither the cellulase being measured nor the enzymes β-glucosidase) and α-glucosidase used in the assay.

13. A process as claimed in claim 12 wherein the solvent is dimethyl sulphoxide.
